(19) Europäisches Patentamt
European Patent Office
Office européen des brevets

(11) Veröffentlichungsnummer: 0 178 587
A2

(12) # EUROPÄISCHE PATENTANMELDUNG

(21) Anmeldenummer: 85112827.2

(22) Anmeldetag: 10.10.85

(51) Int. Cl.[4]: C 07 D 249/08
C 07 D 233/60, A 01 N 43/653
A 01 N 43/50, C 07 D 401/12

(30) Priorität: 17.10.84 DE 3437919

(43) Veröffentlichungstag der Anmeldung:
23.04.86 Patentblatt 86/17

(84) Benannte Vertragsstaaten:
AT BE CH DE FR GB IT LI NL SE

(71) Anmelder: BASF Aktiengesellschaft
Carl-Bosch-Strasse 38
D-6700 Ludwigshafen(DE)

(72) Erfinder: Roeser, Karl, Dr.
Muldweg 11
D-6945 Hirschberg(DE)

(72) Erfinder: Buschmann, Ernst, Dr.
Georg-Ludwig-Krebs-Strasse 10
D-6700 Ludwigshafen(DE)

(72) Erfinder: Ammermann, Eberhard, Dr.
Sachsenstrasse 3
D-6700 Ludwigshafen(DE)

(72) Erfinder: Pommer, Ernst-Heinrich, Dr.
Berliner Platz 7
D-6703 Limburgerhof(DE)

(72) Erfinder: Sauter, Hubert, Dr.
Neckarpromenade 20
D-6800 Mannheim 1(DE)

(54) **Benzyloxyalkylazole und diese enthaltende Fungizide.**

(57) Benzyloxyalkylazole der allgemeinen Formel

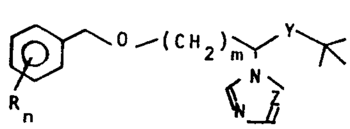

in der

R für Halogen, Alkyl, Alkoxy, Halogenalkyl, gegebenenfalls substituiertes Aryl, gegebenenfalls substituiertes Pyridyl, Aralkyl, Alkylthio oder Wasserstoff steht,

n eine ganze Zahl von 1 bis 5 bedeutet,

m eine ganze Zahl von 1 bis 8 bedeutet,

Y für die Gruppen C=O und HCOH steht und

Z eine CH-Gruppe oder ein N-Atom ist und diese enthaltende Fungizide.

0178587

## Benzyloxyalkylazole und diese enthaltende Fungizide

Die vorliegende Erfindung betrifft neue Benzyloxyalkylazole, diese enthaltende Fungizide und Verfahren zu ihrer Herstellung.

Es ist bekannt, Azolverbindungen z.B. das 2,2-Dimethyl-4-(1,2,4-triazolyl-1)-5-phenyl-pentanon-3 als Fungizide zu verwenden (DE-OS 2 638 470). Eine Aufgabe der vorliegenden Erfindung ist es, neue Verbindungen und Fungizide zu entwickeln, deren Wirksamkeit, insbesondere bei niedrigen Konzentrationen, den bekannten Verbindungen überlegen ist oder die beim Auftreten von Resistenzerscheinungen alternativ angewendet werden können.

Es wurde gefunden, daß Benzyloxyalkylazolverbindungen der Formel I

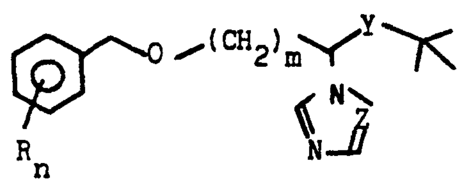

I

in der

R für Halogen, Alkyl, Alkoxy, Halogenalkyl, gegebenenfalls substituiertes Aryl, gegebenenfalls substituiertes Pyridyl, Aralkyl, Alkylthio oder Wasserstoff steht

n eine ganze Zahl von 1 bis 5 bedeutet,

m eine ganze Zahl von 1 bis 8 bedeutet,

Y für die Gruppen C=O und HCOH steht und

Z eine CH-Gruppe oder ein N-Atom bedeutet, eine sehr gute fungizide Wirkung haben.

Bevorzugt werden solche Verbindungen, in denen

R für Halogen, niederes Alkyl mit 1 bis 4 Kohlenstoffatomen, Halogenalkyl mit einem bis zwei Kohlenstoffatomen und 1 bis 3 Halogenatomen, sowie für Wasserstoff steht.

In der Formel I bedeuten beispielsweise

R Halogen (Fluor, Chlor, Brom oder Jod), wobei Fluor, Brom und Chlor bevorzugt werden und insbesondere die Substitutionsmuster 4-Chlor-, 4-Fluor-, 2-Chlor-, 2-Fluor-, 3-Fluor-, 2-Brom-, 4-Brom- und 2,4-Dichlor genannt werden,

weiterhin bedeutet R niedere Alkylreste mit 1 bis 4 Kohlenstoffatomen vorzugsweise Methyl, Ethyl, n-Propyl, n-Butyl und tert.-Butyl, wobei besonders Methyl und tert.-Butyl genannt werden;

Sws/Rei

weiterhin bedeutet R Alkoxyreste mit einem bis zwei Kohlenstoffatomen im Alkylteil, vorzugsweise Methoxy;

außerdem bedeutet R Halogenalkylreste, vor allem solche mit 1 bis 2 Kohlenstoffatomen im Alkylteil und mit 1 bis 5, vorzugsweise 1 bis 3 Halogenatomen, wobei als Halogene Fluor, Chlor und Brom genannt werden und Fluor und Chlor als Halogene bevorzugt werden. Als Beispiel für Halogenmethyl wird Trifluormethyl genannt.

Weiterhin kann R für gegebenenfalls substituiertes Aryl stehen, wobei als Arylrest ggf. durch Halogen substituiertes Phenyl genannt wird und Phenyl, 4-Chlorphenyl- und 4-Fluorphenyl bevorzugt sind. Schließlich kann R auch für gegebenenfalls substituiertes Pyridyl, insbesondere gegebenenfalls halogensubstituiertes Pyridyl, Aralkyl (Benzyl), Alkylthio (mit 1 bis 4 C-Atomen, z.B. Methylthio) oder Wasserstoff stehen.

n kann ganze Zahlen von eins bis fünf bedeuten, wobei die Zahlen 1, 2, 3, 4 und 5 genannt werden und die Zahlen eins und zwei bevorzugt sind.

m bedeutet ganze Zahlen von eins bis acht, wobei die Zahlen 2,3,4,5 und 6 bevorzugt werden.

Y bedeutet eine C=O-Gruppe oder eine HCOH-Gruppe.

Z steht in der Formel I für ein N-Atom oder die Gruppe C-H.

Die Verbindungen der Formel I lassen sich herstellen, indem man einem der im folgenden angegebenen Schemata folgt:

Schema A (Vorprodukt)

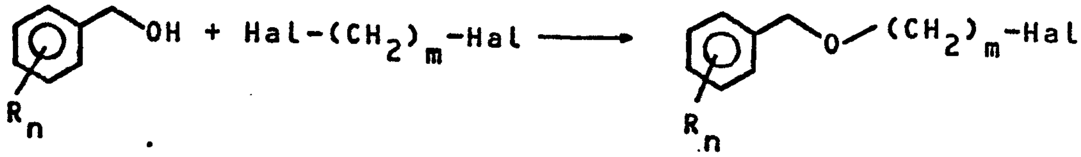

Schema B (Vorprodukt)

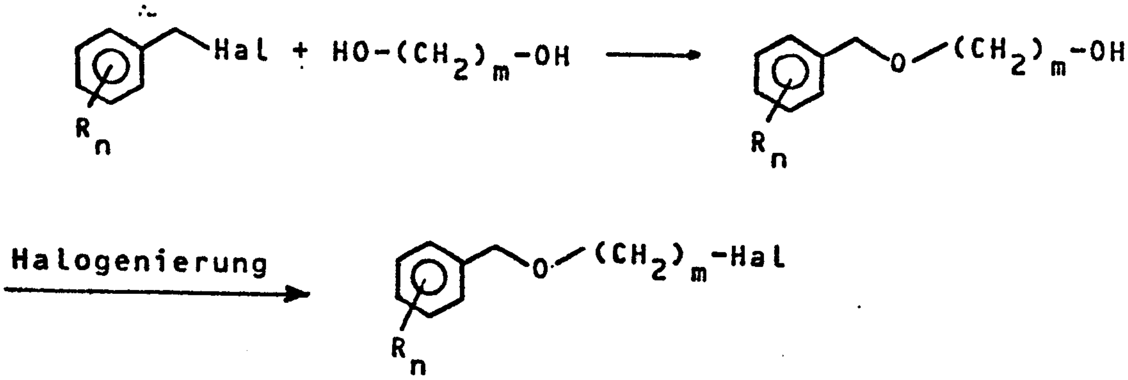


Schema C (Vorprodukt)

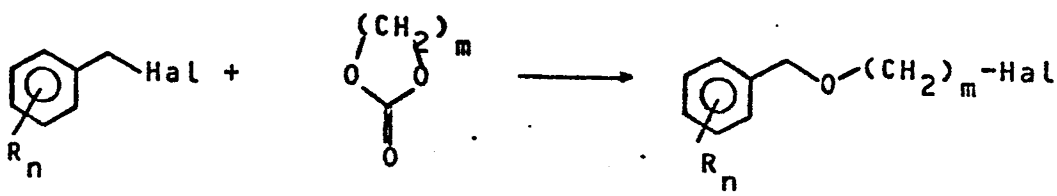

Schema D

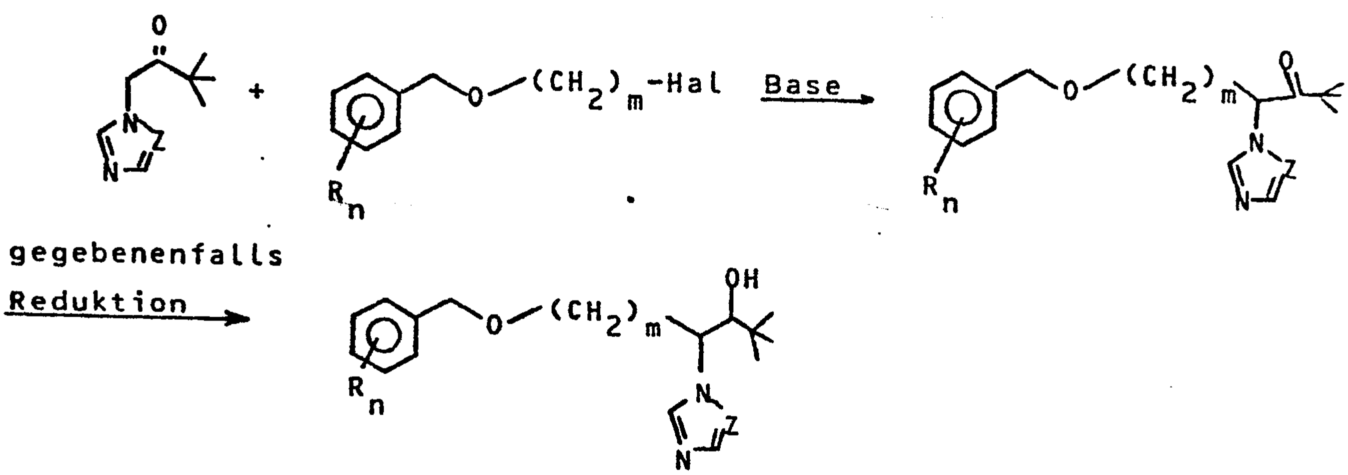

Die Ausgangsverbindungen der Formel II lassen sich nach an sich bekannten Verfahren herstellen, indem man

a) das Monoalkalisalz eines alpha,omega-Glycols mit einem entsprechenden Benzylhalogenid alkyliert und den so erhaltenen Alkohol z.B. mittels N-Bromsuccinimid/Triphenylphosphin, in das Bromid überführt (Schema B),

b) oder indem man ein Phenol mit einem alpha,omega-Dihalogenalkan alkyliert (Schema A).

Auf diese Weise lassen sich die folgenden Zwischenprodukte darstellen:

| $R_n$ | m | X | Phys. Daten Kp |
|---|---|---|---|
| 4-Cl | 2 | Cl | |
| 4-Cl | 2 | Br | 116°C/0,1 mb. |
| 4-Cl | 3 | Cl | 108°C/0,4 mb. |
| 4-Cl | 3 | Br | |
| 4-Cl | 4 | Cl | |
| 4-Cl | 4 | Br | |
| 4-Cl | 5 | Cl | 155 - 170°C/0,4 mb. |
| 4-Cl | 5 | Br | |
| 4-Cl | 6 | Cl | |
| 4-Cl | 6 | Br | |
| 4-Cl | 7 | Cl | |
| 4-Cl | 7 | Br | |
| 2-Cl | 2 | Cl | |
| 2-Cl | 2 | Br | |
| 2-Cl | 3 | Cl | |
| 2-Cl | 3 | Br | |
| 2-Cl | 4 | Cl | |
| 2-Cl | 4 | Br | |
| 2-Cl | 5 | Cl | |
| 2-Cl | 5 | Br | |
| 2-Cl | 6 | Cl | |
| 2-Cl | 6 | Br | |
| 2-Cl | 7 | Cl | |
| 2-Cl | 7 | Br | |
| 2,4-Dichlor | 2 | Cl | 111°C/0,5 mb. |
| 2,4-Dichlor | 2 | Br | |
| 2,4-Dichlor | 3 | Cl | |
| 2,4-Dichlor | 3 | Br | |
| 2,4-Dichlor | 4 | Cl | 140°C/0,5 mb. |
| 2,4-Dichlor | 4 | Br | |
| 2,4-Dichlor | 5 | Cl | |
| 2,4-Cichlor | 5 | Br | |
| 2,4-Dichlor | 6 | Cl | |
| 2,4-Dichlor | 6 | Br | |
| 2,4-Dichlor | 7 | Cl | |
| 2,4-Dichlor | 7 | Br | |
| 4-tert.-Butyl | 2 | Cl | |
| 4-tert.-Butyl | 2 | Br | 112 - 119°C/0,3 mb. |
| 4-tert.-Butyl | 3 | Cl | 140°C/0,5 mb. |
| 4-tert.-Butyl | 3 | Br | |
| 4-tert.-Butyl | 4 | Cl | 148°C/0,5 mb. |

| $R_n$ | m | X | Phys. Daten Kp |
|---|---|---|---|
| 4-tert.-Butyl | 4 | Br | |
| 4-tert.-Butyl | 5 | Cl | 155°C/0,4 mb. |
| 4-tert.-Butyl | 5 | Br | |
| 4-tert.-Butyl | 6 | Cl | 160°C/0,4 mb. |
| 4-tert.-Butyl | 6 | Br | |
| 4-tert.-Butyl | 7 | Cl | |
| 4-tert.-Butyl | 7 | Br | |
| 4-F | 2 | Cl | |
| 4-F | 2 | Br | 88°C/0,5 mb. |
| 4-F | 3 | Cl | |
| 4-F | 3 | Br | |
| 4-F | 4 | Cl | |
| 4-F | 4 | Br | |
| 4-F | 5 | Cl | |
| 4-F | 5 | Br | |
| 4-F | 6 | Cl | |
| 4-F | 6 | Br | |
| 4-F | 7 | Cl | |
| 4-F | 7 | Br | |
| 2,4,6-Trichlor | 2 | Cl | |
| 2,4,6-Trichlor | 2 | Br | |
| 2,4,6-Trichlor | 3 | Cl | |
| 2,4,6-Trichlor | 3 | Br | |
| 2,4,6-Trichlor | 4 | Cl | |
| 2,4,6-Trichlor | 4 | Br | |
| 2,4,6-Trichlor | 5 | Cl | |
| 2,4,6-Trichlor | 5 | Br | |
| 2,4,6-Trichlor | 6 | Cl | |
| 2,4,6-Trichlor | 6 | Br | |
| 2,4,6-Trichlor | 7 | Cl | |
| 2,4,6-Trichlor | 7 | Br | |
| 4-Methyl | 2 | Cl | |
| 4-Methyl | 2 | Br | 94°C/0,4 mb. |
| 4-Methyl | 3 | Cl | 95°C/0,5 mb. |
| 4-Methyl | 3 | Br | |
| 4-Methyl | 4 | Cl | |
| 4-Methyl | 4 | Br | |
| 4-Methyl | 5 | Cl | |
| 4-Methyl | 5 | Br | |
| 4-Methyl | 6 | Cl | |
| 4-Methyl | 6 | Br | |

| $R_n$ | m | X | Phys. Daten Kp |
|---|---|---|---|
| 4-Methyl | 7 | Cl | |
| 4-Methyl | 7 | Br | |
| 4-Br | 2 | Br | 123 - 135°C/0,3 mb. |
| 3-$CH_3$ | 2 | Br | 92°C/0,4 mb. |
| 2-$CF_3$ | 2 | Br | 88°C/0,7 mb. |
| 2-Br | 2 | Br | 116 - 125°C/0,4 mb. |
| 3-F | 2 | Br | 80°C/0,5 mb. |
| 4-$CF_3$ | 3 | Cl | 95°C/0,4 mb. |
| 4-$CF_3$ | 4 | Cl | 125°C/0,5 mb. |
| 4-$CF_3$ | 5 | Cl | 140°C/2,5 mb. |
| 4-$CF_3$ | 6 | Cl | 130°C/0,5 mb. |

Die Verbindungen der allgemeinen Formel I enthalten mindestens ein Asymetriezentrum; im Falle von Y=CHOH enthalten sie mindestens zwei Asymetriezentren und können deshalb als Enantiomeren- und/oder Diastereomerengemische vorliegen. Bei der erfindungsgemäßen Synthese fallen die Verbindungen gewöhnlich als Stereoisomerengemische an; aus diesen können die Isomeren durch geeignete Trennoperationen in reiner Form erhalten werden. Die vorliegende Erfindung umfaßt sowohl die einzelnen Isomeren als auch ihre Gemische.

Durch gezielte Synthese, zum Beispiel durch Reduktion der neuen Ketone der Formel I mit Hilfe von geeigneten Reduktionsmitteln, lassen sich die Isomeren der neuen Alkohole der Formel I auch direkt herstellen.

Herstellungsbeispiel 1

4,2 g (0,14 mol) einer 80 %igen Suspension von NaH in Paraffin wurden in 100 ml trockenem DMF (Dimethylformamid) suspendiert. Dazu tropfte man 23,4 g (0,14 mol) 1-(1-(1,2,4-Triazolyl)-3,3-dimethylbutan-3-on, gelöst in 50 ml trockenem DMF. Nach Abklingen der Gasentwicklung rührte man noch 1 Stunde nach und tropfte dann eine Lösung von 21 g (0,14 mol) 3-Benzyloxy-1-brompropan, gelöst in 50 ml trockenem DMF, zu. Man rührte 4 Stunden bei 70°C nach, ließ erkalten, goß in eine Mischung aus je 500 ml Wasser und Methylenchlorid, trennte die Phasen, extrahierte die wäßrige Phase mit Methylenchlorid, trocknete die vereinigten organischen Phasen und verdampfte das Lösungsmittel unter vermindertem Druck. Das Rohprodukt wurde destilliert; man erhält 22,6 g (51 % d. Th.) 7-Benzyloxy-2,2--dimethyl-4(1-(1,2,4-triazolyl)-heptan-3-on. IR: 1717, 1502, 1276, 1139, 1103 (Verbindung Nr. 1).

Herstellbeispiel 2

15,0 g (0,048 mol) der Verbindung Nr. 1 wurden in 50 ml Isopropanol gelöst und mit einer Lösung von 0,61 g (0,016 mol) Natriumborhydrid in Isopropanol versetzt. Man rührte über Nacht bei Raumtemperatur, goß danach auf Wasser, extrahierte die wäßrige Phase mit Essigsäureethylester, trocknete die Extrakte und verdampfte das Lösungsmittel unter vermindertem Druck. Der Rückstand wurde destilliert; man erhält so 12,0 g (80 % d. Th.) des Produktes Nr. 2 (7-Benzyloxy-2,2-dimethyl-4(1-(1,2,4-triazolyl)-heptan-3-ol). IR: 2956, 2869, 1138, 1100, 1075

Auf entsprechende Weise lassen sich die nachstehend aufgelisteten Verbindungen herstellen. Soweit die Substanzen nicht durch physikalische Daten charakterisiert sind, dienen sie dazu, die Breite der Synthesemöglichkeiten darzustellen. A und B sind die beiden Diastereomerengemische.

| Nr. | $R_n$ | m | Y | Z | Phys. Daten IR-Banden |
|---|---|---|---|---|---|
| 3 | H | 2 | C=O | N | |
| 4 | H | 2 | C=O | CH | |
| 5 | H | 2 | HCOH (A) | N | |
| 6 | H | 2 | HCOH (A) | CH | |
| 7 | H | 2 | HCOH (B) | N | |
| 8 | H | 2 | HCOH (B) | CH | |
| 9 | H | 3 | C=O | CH | |
| 10 | H | 3 | HCOH (A) | CH | 1496, 1123, 1113, 1080, 742 |
| 11 | H | 3 | HCOH (A) | N | 2956, 2869, 1275, 1136, 1101 |
| 12 | H | 3 | HCOH (B) | CH | 2953, 2868, 1100, 1081, 745 |
| 13 | H | 4 | C=O | N | 2935, 2860, 1716, 1501, 1102 |
| 14 | H | 4 | C=O | CH | |
| 15 | H | 4 | HCOH (A) | N | 2954, 2867, 1275, 1136, 1102 |
| 16 | H | 4 | HCOH (A) | CH | |
| 17 | H | 4 | HCOH (B) | N | 2965, 2946, 1517, 1141, 699 |
| 18 | H | 4 | HCOH (B) | CH | |
| 19 | H | 5 | C=O | N | |
| 20 | H | 5 | C=O | CH | |
| 21 | H | 5 | HCOH (A) | N | |
| 22 | H | 5 | HCOH (A) | CH | |
| 23 | H | 5 | HCOH (B) | N | |
| 24 | H | 5 | HCOH (B) | CH | |
| 25 | H | 6 | C=O | N | |
| 26 | H | 6 | C=O | CH | |
| 27 | H | 6 | HCOH (A) | N | |
| 28 | H | 6 | HCOH (A) | CH | |
| 29 | H | 6 | HCOH (B) | N | 2963, 2939, 2859, 1518, 1142 |
| 30 | H | 6 | NCOH (B) | CH | |
| 31 | H | 7 | C=O | N | |
| 32 | H | 7 | C=O | CH | |
| 33 | H | 7 | HCOH (A) | N | |
| 34 | H | 7 | HCOH (A) | CH | |
| 35 | H | 7 | HCOH (B) | N | |
| 36 | H | 7 | HCOH (B) | CH | |
| 37 | H | 8 | C=O | N | |
| 38 | H | 8 | C=O | CH | |
| 39 | H | 8 | HCOH (A) | N | |
| 40 | H | 8 | HCOH (A) | CH | |
| 41 | H | 8 | HCOH (B) | N | |
| 42 | H, | 8 | CHOH (B) | CH | |
| 43 | 2-Cl | 2 | C=O | N | |

| Nr. | $R_n$ | m | Y | Z | Phys. Daten | IR-Banden |
|---|---|---|---|---|---|---|
| 44 | 2-Cl | 2 | C=O | CH | | |
| 45 | 2-Cl | 2 | HCOH (A) | N | | |
| 46 | 2-Cl | 2 | HCOH (A) | CH | | |
| 47 | 2-Cl | 2 | HCOH (B) | N | | |
| 48 | 2-Cl | 2 | HCOH (B) | CH | | |
| 49 | 2-Cl | 3 | C=O | N | | |
| 50 | 2-Cl | 3 | C=O | CH | | |
| 51 | 2-Cl | 3 | HCOH (A) | N | | |
| 52 | 2-Cl | 3 | HCOH (A) | CH | | |
| 53 | 2-Cl | 3 | HCOH (B) | N | | |
| 54 | 2-Cl | 3 | HCOH (B) | CH | | |
| 55 | 2-Cl | 4 | C=O | N | | |
| 56 | 2-Cl | 4 | C=O | CH | | |
| 57 | 2-Cl | 4 | HCOH (A) | N | | |
| 58 | 2-Cl | 4 | HCOH (A) | CH | | |
| 59 | 2-Cl | 4 | HCOH (B) | N | | |
| 60 | 2-Cl | 4 | HCOH (B) | CH | | |
| 61 | 2-Cl | 5 | C=O | N | | |
| 62 | 2-Cl | 5 | C=O | CH | | |
| 63 | 2-Cl | 5 | HCOH (A) | N | | |
| 64 | 2-Cl | 5 | HCOH (A) | CH | | |
| 65 | 2-Cl | 5 | HCOH (B) | N | | |
| 66 | 2-Cl | 5 | HCOH (B) | CH | | |
| 67 | 2-Cl | 6 | C=O | N | | |
| 68 | 2-Cl | 6 | C=O | CH | | |
| 69 | 2-Cl | 6 | HCOH (A) | N | | |
| 70 | 2-Cl | 6 | HCOH (A) | CH | | |
| 71 | 2-Cl | 6 | HCOH (B) | N | | |
| 72 | 2-Cl | 6 | HCOH (B) | CH | | |
| 73 | 2-Cl | 7 | C=O | N | | |
| 74 | 2-Cl | 7 | C=O | CH | | |
| 75 | 2-Cl | 7 | HCOH (A) | N | | |
| 76 | 2-Cl | 7 | HCOH (A) | CH | | |
| 77 | 2-Cl | 7 | HCOH (B) | N | | |
| 78 | 2-Cl | 7 | HCOH (B) | CH | | |
| 79 | 2-Cl | 8 | C=O | N | | |
| 80 | 2-Cl | 8 | C=O | CH | | |
| 81 | 2-Cl | 8 | HCOH (A) | N | | |
| 82 | 2-Cl | 8 | HCOH (A) | CH | | |
| 83 | 2-Cl | 8 | HCOH (B) | N | | |
| 84 | 2-Cl | 8 | HCOH (B) | CH | | |

| Nr. | $R_n$ | m | Y | Z | Phys. Daten IR-Banden |
|---|---|---|---|---|---|
| 85 | 2,4-$Cl_2$ | 2 | C=O | N | |
| 86 | 2,4-$Cl_2$ | 2 | C=O | CH | |
| 87 | 2,4-$Cl_2$ | 2 | HCOH (A) | N | Kp = 220°C/0.15 mb. |
| 88 | 2,4-$Cl_2$ | 2 | HCOH (A) | CH | |
| 89 | 2,4-$Cl_2$ | 2 | HCOH (B) | N | |
| 90 | 2,4-$Cl_2$ | 2 | HCOH (B) | CH | |
| 91 | 2,4-$Cl_2$ | 3 | C=O | N | |
| 92 | 2,4-$Cl_2$ | 3 | C=O | CH | |
| 93 | 2,4-$Cl_2$ | 3 | HCOH (A) | N | |
| 94 | 2,4-$Cl_2$ | 3 | HCOH (A) | CH | |
| 95 | 2,4-$Cl_2$ | 3 | HCOH (B) | N | |
| 96 | 2,4-$Cl_2$ | 3 | HCOH (B) | CH | |
| 97 | 2,4-$Cl_2$ | 4 | C=O | N | |
| 98 | 2,4-$Cl_2$ | 4 | C=O | CH | |
| 99 | 2,4-$Cl_2$ | 4 | HCOH (A) | N | 2955, 2868, 1473, 1135, 1096 |
| 100 | 2,4-$Cl_2$ | 4 | HCOH (A) | CH | |
| 101 | 2,4-$Cl_2$ | 4 | HCOH (B) | N | |
| 102 | 2,4-$Cl_2$ | 4 | HCOH (B) | CH | |
| 103 | 2,4-$Cl_2$ | 5 | C=O | N | |
| 104 | 2,4-$Cl_2$ | 5 | C=O | CH | |
| 105 | 2,4-$Cl_2$ | 5 | HCOH (A) | N | |
| 106 | 2,4-$Cl_2$ | 5 | HCOH (A) | CH | |
| 107 | 2,4-$Cl_2$ | 5 | HCOH (B) | N | |
| 108 | 2,4-$Cl_2$ | 5 | HCOH (B) | CH | |
| 109 | 2,4-$Cl_2$ | 5 | HCOH (B) | N | |
| 110 | 2,4-$Cl_2$ | 6 | C=O | CH | |
| 111 | 2,4-$Cl_2$ | 6 | HCOH (A) | N | |
| 112 | 2,4-$Cl_2$ | 6 | HCOH (A) | CH | |
| 113 | 2,4-$Cl_2$ | 6 | HCOH (B) | N | |
| 114 | 2,4-$Cl_2$ | 6 | HCOH (B) | CH | |
| 115 | 2,4-$Cl_2$ | 7 | C=O | N | |
| 116 | 2,4-$Cl_2$ | 7 | C=O | CH | |
| 117 | 2,4-$Cl_2$ | 7 | HCOH (A) | N | |
| 118 | 2,4-$Cl_2$ | 7 | HCOH (A) | CH | |
| 119 | 2,4-$Cl_2$ | 7 | HCOH (B) | N | |
| 120 | 2,4-$Cl_2$ | 7 | HCOH (B) | CH | |
| 121 | 2,4-$Cl_2$ | 8 | C=O | N | |
| 122 | 2,4-$Cl_2$ | 8 | C=O | CH | |
| 123 | 2,4-$Cl_2$ | 8 | HCOH (A) | N | |
| 124 | 2,4-$Cl_2$ | 8 | HCOH (A) | CH | |
| 125 | 2,4-$Cl_2$ | 8 | HCOH (B) | N | |

| Nr. | $R_n$ | m | Y | Z | Phys. Daten IR-Banden |
|---|---|---|---|---|---|
| 126 | 2,4-$Cl_2$ | 8 | HCOH (B) | CH | |
| 127 | 4-Cl | 2 | C=O | N | 1717, 1502, 1492, 1090, 1015 |
| 128 | 4-Cl | 2 | C=O | CH | |
| 129 | 4-Cl | 2 | HCOH (A) | N | 1134, 1123, 1097, 1087, 1014 |
| 130 | 4-Cl | 2 | HCOH (A) | CH | |
| 131 | 4-Cl | 2 | HCOH (B) | N | |
| 132 | 4-Cl | 2 | HCOH (B) | CH | |
| 133 | 4-Cl | 3 | C=O | N | 1717, 1492, 1097, 1089, 1015 |
| 134 | 4-Cl | 3 | C=O | CH | 1714, 1492, 1108, 1096, 1087 |
| 135 | 4-Cl | 3 | HCOH (A) | N | 2956, 1275, 1136, 1093, 1015 |
| 136 | 4-Cl | 3 | HCOH (A) | CH | |
| 137 | 4-Cl | 3 | HCOH (B) | N | 3320, 2963, 1510, 1146, 809 |
| 138 | 4-Cl | 3 | HCOH (B) | CH | |
| 139 | 4-Cl | 4 | C=O | N | |
| 140 | 4-Cl | 4 | C=O | CH | |
| 141 | 4-Cl | 4 | HCOH (A) | N | |
| 142 | 4-Cl | 4 | HCOH (A) | CH | |
| 143 | 4-Cl | 4 | HCOH (B) | N | |
| 144 | 4-Cl | 4 | HCOH (B) | CH | |
| 145 | 4-Cl | 5 | C=O | N | 2936, 2863, 1716, 1492, 1090 |
| 146 | 4-Cl | 5 | C=O | CH | |
| 147 | 4-Cl | 5 | HCOH (A) | N | 2939, 2863, 1492, 1090, 1015 |
| 148 | 4-Cl | 5 | HCOH (A) | CH | |
| 149 | 4-Cl | 5 | HCOH (B) | N | 2952, 2935, 2867, 2852, 813 |
| 150 | 4-Cl | 5 | HCOH (B) | CH | |
| 151 | 4-Cl | 6 | C=O | N | |
| 152 | 4-Cl | 6 | C=O | CH | |
| 153 | 4-Cl | 6 | HCOH (A) | N | |
| 154 | 4-Cl | 6 | HCOH (A) | CH | |
| 155 | 4-Cl | 6 | HCOH (B) | N | |
| 156 | 4-Cl | 6 | HCOH (B) | CH | |
| 157 | 4-Cl | 7 | C=O | N | |
| 158 | 4-Cl | 7 | C=O | CH | |
| 159 | 4-Cl | 7 | HCOH (A) | N | |
| 160 | 4-Cl | 7 | HCOH (A) | CH | |
| 161 | 4-Cl | 7 | HCOH (B) | N | |
| 162 | 4-Cl | 7 | HCOH (B) | CH | |
| 163 | 4-Cl | 8 | C=O | N | |
| 164 | 4-Cl | 8 | C=O | CH | |
| 165 | 4-Cl | 8 | HCOH (A) | N | |
| 166 | 4-Cl | 8 | HCOH (A) | CH | |

| Nr. | $R_n$ | m | Y | Z | Phys. Daten IR-Banden |
|---|---|---|---|---|---|
| 167 | 4-Cl | 8 | HCOH (B) | N | |
| 168 | 4-Cl | 8 | HCOH (B) | CH | |
| 169 | 4-$OCH_3$ | 2 | C=O | N | |
| 170 | 4-$OCH_3$ | 2 | C=O | CH | |
| 171 | 4-$OCH_3$ | 2 | HCOH (A) | N | |
| 172 | 4-$OCH_3$ | 2 | HCOH (A) | CH | |
| 173 | 4-$OCH_3$ | 2 | HCOH (B) | N | |
| 174 | 4-$OCH_3$ | 2 | HCOH (B) | CH | |
| 175 | 4-$OCH_3$ | 3 | C=O | N | |
| 176 | 4-$OCH_3$ | 3 | C=O | CH | |
| 177 | 4-$OCH_3$ | 3 | HCOH (A) | N | |
| 178 | 4-$OCH_3$ | 3 | HCOH (A) | CH | |
| 179 | 4-$OCH_3$ | 3 | HCOH (B) | N | |
| 180 | 4-$OCH_3$ | 3 | HCOH (B) | CH | |
| 181 | 4-$OCH_3$ | 4 | C=O | N | |
| 182 | 4-$OCH_3$ | 4 | C=O | CH | |
| 183 | 4-$OCH_3$ | 4 | HCOH (A) | N | |
| 184 | 4-$OCH_3$ | 4 | HCOH (A) | CH | |
| 185 | 4-$OCH_3$ | 4 | HCOH (B) | N | |
| 186 | 4-$OCH_3$ | 4 | HCOH (B) | CH | |
| 187 | 4-$OCH_3$ | 5 | C=O | N | |
| 188 | 4-$OCH_3$ | 5 | C=O | CH | |
| 189 | 4-$OCH_3$ | 5 | HCOH (A) | N | |
| 190 | 4-$OCH_3$ | 5 | HCOH (A) | CH | |
| 191 | 4-$OCH_3$ | 5 | HCOH (B) | N | |
| 192 | 4-$OCH_3$ | 5 | HCOH (B) | CH | |
| 193 | 4-$OCH_3$ | 6 | C=O | N | |
| 194 | 4-$OCH_3$ | 6 | C=O | CH | |
| 195 | 4-$OCH_3$ | 6 | HCOH (A) | N | |
| 196 | 4-$OCH_3$ | 6 | HCOH (A) | CH | |
| 197 | 4-$OCH_3$ | 6 | HCOH (B) | N | |
| 198 | 4-$OCH_3$ | 6 | HCOH (B) | CH | |
| 199 | 4-$OCH_3$ | 7 | C=O | N | |
| 200 | 4-$OCH_3$ | 7 | C=O | CH | |
| 201 | 4-$OCH_2$ | 7 | HCOH (A) | N | |
| 202 | 4-$OCH_3$ | 7 | HCOH (A) | CH | |
| 203 | 4-$OCH_3$ | 7 | HCOH (B) | N | |
| 204 | 4-$OCH_3$ | 7 | HCOH (B) | CH | |
| 205 | 4-$OCH_3$ | 8 | C=O | N | |
| 206 | 4-$OCH_3$ | 8 | C=O | CH | |
| 207 | 4-$OCH_3$ | 8 | HCOH (A) | N | |

| Nr. | $R_n$ | m | Y | Z | Phys. Daten IR-Banden |
|---|---|---|---|---|---|
| 208 | 4-$OCH_3$ | 8 | HCOH (A) | CH | |
| 209 | 4-$OCH_3$ | 8 | HCOH (B) | N | |
| 210 | 4-$OCH_3$ | 8 | HCOH (B) | CH | |
| 211 | 4-$CF_3$ | 2 | C=O | N | 1718, 1327, 1164, 1124, 1067 |
| 212 | 4-$CF_3$ | 2 | C=O | CH | |
| 213 | 4-$CF_3$ | 2 | HCOH (A) | N | 1326, 1164, 1128, 1125, 1067 |
| 214 | 4-$CF_3$ | 2 | HCOH (A) | CH | |
| 215 | 4-$CF_3$ | 2 | HCOH (B) | N | 1327, 1164, 1124, 1067, 1018 |
| 216 | 4-$CF_3$ | 2 | HCOH (B) | CH | |
| 217 | 4-$CF_3$ | 3 | C=O | N | 1717, 1327, 1164, 1124, 1108 |
| 218 | 4-$CF_3$ | 3 | C=O | CH | |
| 219 | 4-$CF_3$ | 3 | HCOH (A) | N | 1327, 1164, 1125, 1106, 1067 |
| 220 | 4-$CF_3$ | 3 | HCOH (A) | CH | |
| 221 | 4-$CF_3$ | 3 | HCOH (B) | N | 1327, 1168, 1129, 1126, 1104 |
| 222 | 4-$CF_3$ | 3 | HCOH (B) | CH | |
| 223 | 4-$CF_3$ | 4 | C=O | N | 1326, 1124, 1607, 1105, 1164 |
| 224 | 4-$CF_3$ | 4 | C=O | CH | |
| 225 | 4-$CF_3$ | 4 | HCOH (A) | N | 1326, 1164, 1125, 1105, 1067 |
| 226 | 4-$CF_3$ | 4 | HCOH (A) | CH | |
| 227 | 4-$CF_3$ | 4 | HCOH (B) | N | 1328, 1138, 1123, 1099, 1067 |
| 228 | 4-$CF_3$ | 4 | HCOH (B) | CH | |
| 229 | 4-$CF_3$ | 5 | C=O | N | 1327, 1164, 1124, 1105, 1067 |
| 230 | 4-$CF_3$ | 5 | C=O | CH | |
| 231 | 4-$CF_3$ | 5 | HCOH (A) | N | 1236, 1164, 1125, 1106, 1067 |
| 232 | 4-$CF_3$ | 5 | HCOH (A) | CH | |
| 233 | 4-$CF_3$ | 5 | HCOH (B) | N | 1327, 1164, 1125, 1105, 1067 |
| 234 | 4-$CF_3$ | 5 | HCOH (B) | CH | |
| 235 | 4-$CF_3$ | 6 | C=O | N | |
| 236 | 4-$CF_3$ | 6 | C=O | CH | |
| 237 | 4-$CF_3$ | 6 | HCOH (A) | N | 1327, 1164, 1125, 1104, 1067 |
| 238 | 4-$CF_3$ | 6 | HCOH (A) | CH | |
| 239 | 4-$CF_3$ | 6 | HCOH (B) | N | |
| 240 | 4-$CF_3$ | 6 | HCOH (B) | CH | |
| 241 | 4-$CF_3$ | 7 | C=O | N | |
| 242 | 4-$CF_3$ | 7 | C=O | CH | |
| 243 | 4-$CF_3$ | 7 | HCOH (A) | N | |
| 244 | 4-$CF_3$ | 7 | HCOH (A) | CH | |
| 245 | 4-$CF_3$ | 7 | HCOH (B) | N | |
| 246 | 4-$CF_3$ | 7 | HCOH (B) | CH | |
| 247 | 4-$CF_3$ | 8 | C=O | N | |
| 248 | 4-$CF_3$ | 8 | C=O | CH | |

| Nr. | $R_n$ | m | Y | Z | Phys. Daten IR-Banden |
|---|---|---|---|---|---|
| 249 | 4-$CF_3$ | 8 | HCOH (A) | N | |
| 250 | 4-$CF_3$ | 8 | HCOH (A) | CH | |
| 251 | 4-$CF_3$ | 8 | HCOH (B) | N | |
| 252 | 4-$CF_3$ | 8 | HCOH (B) | CH | |
| 253 | 4-$CH_3$ | 2 | C=O | N | 1718, 1502, 1276, 1139, 1096 |
| 254 | 4-$CH_3$ | 2 | C=O | CH | |
| 255 | 4-$CH_3$ | 2 | HCOH (A) | N | 1134, 1123, 1111, 1098, 809 |
| 256 | 4-$CH_3$ | 2 | HCOH (A) | CH | |
| 257 | 4-$CH_3$ | 2 | HCOH (B) | N | |
| 258 | 4-$CH_3$ | 2 | HCOH (B) | CH | |
| 259 | 4-$CH_3$ | 3 | C=O | N | 2965, 2933, 2867, 1717, 1100 |
| 260 | 4-$CH_3$ | 3 | C=O | CH | |
| 261 | 4-$CH_3$ | 3 | HCOH (A) | N | |
| 262 | 4-$CH_3$ | 3 | HCOH (A) | CH | |
| 263 | 4-$CH_3$ | 3 | HCOH (B) | N | 2956, 2868, 1504, 1138, 1099 |
| 264 | 4-$CH_3$ | 3 | HCOH (B) | CH | |
| 265 | 4-$CH_3$ | 4 | C=O | N | |
| 266 | 4-$CH_3$ | 4 | C=O | CH | |
| 267 | 4-$CH_3$ | 4 | HCOH (A) | N | |
| 268 | 4-$CH_3$ | 4 | HCOH (A) | CH | |
| 269 | 4-$CH_3$ | 4 | HCOH (B) | N | |
| 270 | 4-$CH_3$ | 4 | HCOH (B) | CH | |
| 271 | 4-$CH_3$ | 5 | C=O | N | |
| 272 | 4-$CH_3$ | 5 | C=O | CH | |
| 273 | 4-$CH_3$ | 5 | HCOH (A) | N | |
| 274 | 4-$CH_3$ | 5 | HCOH (A) | CH | |
| 275 | 4-$CH_3$ | 5 | HCOH (B) | N | |
| 276 | 4-$CH_3$ | 5 | HCOH (B) | CH | |
| 277 | 4-$CH_3$ | 6 | C=O | N | |
| 278 | 4-$CH_3$ | 6 | C=O | CH | |
| 279 | 4-$CH_3$ | 6 | HCOH (A) | N | |
| 280 | 4-$CH_3$ | 6 | HCOH (A) | CH | |
| 281 | 4-$CH_3$ | 6 | HCOH (B) | N | |
| 282 | 4-$CH_3$ | 6 | HCOH (B) | CH | |
| 283 | 4-$CH_3$ | 7 | C=O | N | |
| 284 | 4-$CH_3$ | 7 | C=O | CH | |
| 285 | 4-$CH_3$ | 7 | HCOH (A) | N | |
| 286 | 4-$CH_3$ | 7 | HCOH (A) | CH | |
| 287 | 4-$CH_3$ | 7 | HCOH (B) | N | |
| 288 | 4-$CH_3$ | 7 | HCOH (B) | CH | |
| 289 | 4-$CH_3$ | 8 | C=O | N | |

0178587

| Nr. | $R_n$ | m | Y | Z | Phys. Daten IR-Banden |
|---|---|---|---|---|---|
| 290 | 4-$CH_3$ | 8 | C=O | CH | |
| 291 | 4-$CH_3$ | 8 | HCOH (A) | N | |
| 292 | 4-$CH_3$ | 8 | HCOH (A) | CH | |
| 293 | 4-$CH_3$ | 8 | HCOH (B) | N | |
| 294 | 4-$CH_3$ | 8 | HCOH (B) | CH | |
| 295 | 4-F | 2 | C=O | N | 1717, 1510, 1223, 1111, 1094 |
| 296 | 4-F | 2 | C=O | CH | |
| 297 | 4-F | 2 | HCOH (A) | N | Fp = 75-80°C |
| 298 | 4-F | 2 | HCOH (A) | CH | |
| 299 | 4-F | 2 | HCOH (B) | N | |
| 300 | 4-F | 2 | HCOH (B) | CH | |
| 301 | 4-F | 3 | C=O | N | |
| 302 | 4-F | 3 | C=O | CH | |
| 303 | 4-F | 3 | HCOH (A) | N | |
| 304 | 4-F | 3 | HCOH (A) | CH | |
| 305 | 4-F | 3 | HCOH (B) | N | |
| 306 | 4-F | 3 | HCOH (B) | CH | |
| 307 | 4-F | 4 | C=O | N | |
| 308 | 4-F | 4 | C=O | CH | |
| 309 | 4-F | 4 | HCOH (A) | N | |
| 310 | 4-F | 4 | HCOH (A) | CH | |
| 311 | 4-F | 4 | HCOH (B) | N | |
| 312 | 4-F | 4 | HCOH (B) | CH | |
| 313 | 4-F | 5 | C=O | N | |
| 314 | 4-F | 5 | C=O | CH | |
| 315 | 4-F | 5 | HCOH (A) | N | |
| 316 | 4-F | 5 | HCOH (A) | CH | |
| 317 | 4-F | 5 | HCOH (B) | N | |
| 318 | 4-F | 5 | HCOH (B) | CH | |
| 319 | 4-F | 6 | C=O | N | |
| 320 | 4-F | 6 | C=O | CH | |
| 321 | 4-F | 6 | HCOH (A) | N | |
| 322 | 4-F | 6 | HCOH (A) | CH | |
| 323 | 4-F | 6 | HCOH (B) | N | |
| 324 | 4-F | 6 | HCOH (B) | CH | |
| 325 | 4-F | 7 | C=O | N | |
| 326 | 4-F | 7 | C=O | CH | |
| 327 | 4-F | 7 | HCOH (A) | N | |
| 328 | 4-F | 7 | HCOH (A) | CH | |
| 329 | 4-F | 7 | HCOH (B) | N | |
| 330 | 4-F | 7 | HCOH (B) | CH | |

| Nr. | $R_n$ | m | Y | Z | Phys. Daten IR-Banden |
|---|---|---|---|---|---|
| 331 | 4-F | 8 | C=O | N | |
| 332 | 4-F | 8 | C=O | CH | |
| 333 | 4-F | 8 | HCOH (A) | N | |
| 334 | 4-F | 8 | HCOH (A) | CH | |
| 335 | 4-F | 8 | HCOH (B) | N | |
| 336 | 4-F | 8 | HCOH (B) | CH | |
| 337 | $4-C(CH_3)_3$ | 2 | C=O | N | 2963, 2907, 2869, 1718, 1274, 1110 |
| 338 | $4-C(CH_3)_3$ | 2 | C=O | CH | |
| 339 | $4-C(CH_3)_3$ | 2 | HCOH (A) | N | 2960, 2869, 1186, 1109, 1090 |
| 340 | $4-C(CH_3)_3$ | 2 | HCOH (A) | CH | |
| 341 | $4-C(CH_3)_3$ | 2 | HCOH (B) | N | |
| 342 | $4-C(CH_3)_3$ | 2 | HCOH (B) | CH | |
| 343 | $4-C(CH_3)_3$ | 3 | C=O | N | 2963, 2868, 1717, 1275, 1109 |
| 344 | $4-C(CH_3)_3$ | 3 | C=O | CH | |
| 345 | $4-C(CH_3)_3$ | 3 | HCOH (A) | N | 2959, 2908, 2868, 1108, 1100 |
| 346 | $4-C(CH_3)_3$ | 3 | HCOH (A) | CH | |
| 347 | $4-C(CH_3)_3$ | 3 | HCOH (B) | N | 2961, 2907, 2868, 1363, 1109 |
| 348 | $4-C(CH_3)_3$ | 3 | HCOH (B) | CH | |
| 349 | $4-C(CH_3)_3$ | 4 | C=O | N | 2963, 2868, 1717, 1276, 1109 |
| 350 | $4-C(CH_3)_3$ | 4 | C=O | CH | |
| 351 | $4-C(CH_3)_3$ | 4 | HCOH (A) | N | 2958, 2907, 2867, 1368, 1108 |
| 352 | $4-C(CH_3)_3$ | 4 | HCOH (A) | CH | |
| 353 | $4-C(CH_3)_3$ | 4 | HCOH (B) | N | |
| 354 | $4-C(CH_3)_3$ | 4 | HCOH (B) | CH | |
| 355 | $4-C(CH_3)_3$ | 5 | C=O | N | 2963, 2865, 1717, 1501, 1109 |
| 356 | $4-C(CH_3)_3$ | 5 | C=O | CH | |
| 357 | $4-C(CH_3)_3$ | 5 | HCOH (A) | N | 2959, 2908, 2865, 1363, 1109 |
| 358 | $4-C(CH_3)_3$ | 5 | HCOH (A) | CH | |
| 359 | $4-C(CH_3)_3$ | 5 | HCOH (B) | N | 2959, 1363, 1274, 1109 |
| 360 | $4-C(CH_3)_3$ | 5 | HCOH (B) | CH | |
| 361 | $4-C(CH_3)_3$ | 6 | C=O | N | 2963, 2935, 2861, 1717, 1326 |
| 362 | $4-C(CH_3)_3$ | 6 | C=O | CH | |
| 363 | $4-C(CH_3)_3$ | 6 | HCOH (A) | N | |
| 364 | $4-C(CH_3)_3$ | 6 | HCOH (A) | CH | |
| 365 | $4-C(CH_3)_3$ | 6 | HCOH (B) | N | 2958, 2907, 2867, 1363, 1108 |
| 366 | $4-C(CH_3)_3$ | 6 | HCOH (B) | CH | |
| 367 | $4-C(CH_3)_3$ | 7 | C=O | N | |
| 368 | $4-C(CH_3)_3$ | 7 | C=O | CH | |
| 369 | $4-C(CH_3)_3$ | 7 | HCOH (A) | N | |
| 370 | $4-C(CH_3)_3$ | 7 | HCOH (A) | CH | |
| 371 | $4-C(CH_3)_3$ | 7 | HCOH (B) | N | |

| Nr. | $R_n$ | m | Y | Z | Phys. Daten IR-Banden |
|---|---|---|---|---|---|
| 372 | 4-$C(CH_3)_3$ | 7 | HCOH (B) | CH | |
| 373 | 4-$C(CH_3)_3$ | 8 | C=O | N | |
| 374 | 4-$C(CH_3)_3$ | 8 | C=O | CH | |
| 375 | 4-$C(CH_3)_3$ | 8 | HCOH (A) | N | |
| 376 | 4-$C(CH_3)_3$ | 8 | HCOH (A) | CH | |
| 377 | 4-$C(CH_3)_3$ | 8 | HCOH (B) | N | |
| 378 | 4-$C(CH_3)_3$ | 8 | HCOH (B) | CH | |
| 379 | 2-$CF_3$ | 2 | CO | N | 1718, 1315, 1165, 1122, 1060 |
| 380 | 2-$CF_3$ | 2 | HCOH (A) | N | 1315, 1165, 1123, 1060, 1040 |
| 381 | 2-$CF_3$ | 2 | HCOH (B) | N | 1317, 1178, 1135, 1123, 1090 |
| 382 | 2-Br | 2 | CO | N | 1717, 1502, 1276, 1138, 1107 |
| 383 | 2-Br | 2 | HCOH (A) | N | |
| 384 | 2-Br | 2 | HCOH (B) | N | 2957, 2924, 1138, 1103, 751 |
| 385 | 3-F | 2 | CO | N | 2968, 1717, 1276, 1256, 1139 |
| 386 | 3-$CH_3$ | 2 | CO | N | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Cercospora musae an Bananen,
Pseudocercosporella herpotrichoides an Weizen, Gerste,
Pyricularia oryzae an Reis,
Hemileia vastatrix an Kaffee,
Alternaria solani an Kartoffeln, Tomaten
Plasmopara viticola an Reben sowie Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in

bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle, (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz u.a. zur Bekämpfung holzzerstörender Pilze wie Coniophora puteanea und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung Nr. 11 mit 10 Gewichtsteilen N-Methyl-alpha-pyrrolidon und erhält eine Lösung, die zur Anwendung in Form kleinster Tropfen geeignet ist.

II. 20 Gewichtsteile der Verbindung Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von

40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung Nr. 15 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gewichtsteile der Verbindung Nr. 87 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gewichtsteile der Verbindung Nr. 99 werden den mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

VI. 3 Gewichtsteile der Verbindung Nr. 129 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung Nr. 147 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung Nr. 213 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung Nr. 215 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,

heterocyclische Strukturen, wie
2-Heptadecyl-2-imidazolin-acetat
2,4-Dichlor-6-(o-chloranilino)-s-triazin
O,O-Diethyl-phthalimidophosphonothioat
5-Amino-1-(bis-(dimethylamino)-phosphinyl)-3-phenyl-1,2,4-triazol

2,3-Dicyano-1,4-dithiaanthrachinon
2-Thio-1,3-dithio-(4,5,6)-chinoxalin
1-(Butylcarbamoyl)-2-benzimidazol-carbaminsäuremethylester
2-Methoxycarbonylamino-benzimidazol
2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethylthio-phthalimid
N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Iod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1[2-(2,4-Dichlorphenyl)-4-n-propyl-1,3-dioxolan-2-yl-ethyl]-1-H-1,2,4-
-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
alpha-(2-Chlorphenyl)-alpha-(4-chlorphenyl)-5-pyrimidin-methanol
5-Butyl-2-dimethylamino-4-hyroxy-6-methyl-pyrimidin

Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol

sowie verschiedene Fungizide, wie
Dodecylguanidinacetat
3-(3-(3,5-Dimethyl-2-oxycyclohexyl)-2-hydroxyethyl)-gluataramid
Hexachlorbenzol
DL-Methyl-N-(2,6-dimethyl-phenyl)-N-furoyl(2)-alaninat,
DL-N-(2,6-Dimethyl-phenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl(-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid.
2-Cyano-N-(ethylaminocarbonyl)-2-methoximino)-acetamid
1-(2-(2,4-Dichlorphenyl)-pentyl)-1H-1,2,4-triazol
2,4-Difluor-alpha-(1H-1,2,4-triazolyl-1-methyl)-benzhydrylalkohol

Für die folgenden Versuche wurde als Vergleich der bekannte Wirkstoff 2,2-Dimethyl-4-(1,2,4-triazolyl-1)-5-phenyl-pentanon-3 (A) verwendet.

Anwendungsbeispiel 1

Wirksamkeit gegen Gurkenmehltau

Blätter von in Töpfen gewachsenen Gurkenkeimlingen der Sorte "Chinesische Schlange" werden im Zweiblattstadium mit einer Sporensuspension des Gurkenmehltaus (Erysiphe cichoracearum) besprüht. Nach etwa 20 Stunden werden die Versuchspflanzen mit wäßriger Spritzbrühe, die 80 % Wirkstoff und 20 % Emulgiermittel in der Trockensubstanz enthält, bis zur Tropfnässe besprüht. Nach dem Antrocknen des Spritzbelages werden sie anschließend im Gewächshaus bei Temperaturen zwischen 20 und 22°C und 70 bis 80 % relativer Luftfeuchtigkeit aufgestellt. Zur Beurteilung der Wirksamkeit der neuen Stoffe wird das Ausmaß der Pilzentwicklung nach 21 Tagen ermittelt.

Das Ergebnis des Versuches zeigt, daß beispielsweise die Verbindungen Nr. 2, 11, 15, 87, 99, 129, 147, 213, 215, 231, 233, 255, 339, 351, 365, 379, 380, 381, 383 und 384 bei der Anwendung als 0,025 %ige Spritzbrühe eine bessere fungizide Wirkung zeigen (z.B. 100 %) als der bekannte Wirkstoff A (60 %).

Anwendungsbeispiel 2

Wirksamkeit gegen Botrytis cinerea an Paprika

Paprikasämlinge der Sorte "Neusiedler Ideal Elite" werden, nachdem sich 4 bis 5 Blätter gut entwickelt haben, mit wäßrigen Suspensionen, die 80 % Wirkstoff und 20 % Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß beispielsweise die Verbindungen Nr. 17, 147, 231, 233, 255 und 297 bei der Anwendung als 0,05 %ige Spritzbrühe eine bessere fungizide Wirkung zeigen (z.B. 97 %) als der Wirkstoff A (60 %).

Patentansprüche

1. Benzyloxyalkylazole der allgemeinen Formel I

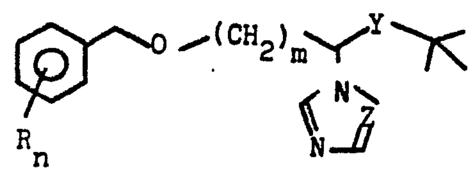

I

in der

R für Halogen, Alkyl, Alkoxy, Halogenalkyl, gegebenenfalls substituiertes Aryl, gegebenenfalls substituiertes Pyridyl, Aralkyl, Alkylthio oder Wasserstoff steht,

n eine ganze Zahl von 1 bis 5 bedeutet,

m eine ganze Zahl von 1 bis 8 bedeutet,

Y für die Gruppen C=O und HCOH steht und

Z eine CH-Gruppe oder ein N-Atom ist.

2. Benzyloxyalkylazolderivate der Formel I gemäß Anspruch 1, wobei R für Wasserstoff, Halogen, niederes Alkyl mit 1 bis 4 Kohlenstoffatomen oder Halogenalkyl mit 1 bis 2 Kohlenstoffatomen und 1 bis 3 Halogenatomen steht.

3. Verfahren zur Herstellung von Verbindungen der Formel I gemäß Anspruch 1, dadurch gekennzeichnet, daß man eine Verbindung der Formel II

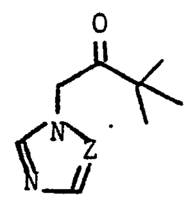

II

in der Z die oben angegebene Bedeutung hat, mit einem Benzyloxyalkylhalogenid der Formel III

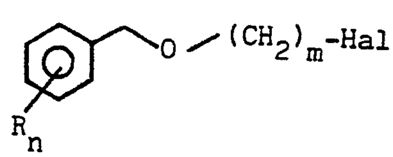

III

in der R, n und m die oben angegebene Bedeutung haben und Hal für die Halogene Chlor, Brom oder Iod steht gegebenenfalls in Gegenwart eines

Lösungs- oder Verdünnungsmittels und gegebenenfalls unter Einwirkung einer Base umsetzt und die so erhaltenen Ketone der Formel I (Y= C=O) gegebenenfalls reduziert.

4. Fungizid, gekennzeichnet durch einen Gehalt an einem Benzyloxyalkylazolderivat der Formel I gemäß Anspruch 1.

5. Verfahren zur Bekämpfung von Pilzen, dadurch gekennzeichnet, daß man ein Benzyloxyalkylazol der allgemeinen Formel I

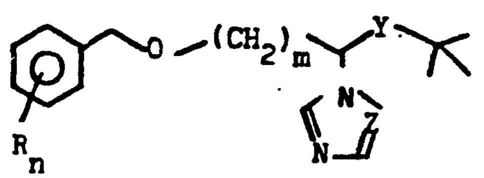

I

in der

R für Halogen, Alkyl, Alkoxy, Halogenalkyl, gegebenenfalls substituiertes Aryl, gegebenenfalls substituiertes Pyridyl, Aralkyl, Alkylthio oder Wasserstoff steht,

n eine ganze Zahl von 1 bis 5 bedeutet,

m eine ganze Zahl von 1 bis 8 bedeutet,

Y für die Gruppen C=O und HCOH steht und

Z eine CH-Gruppe oder ein N-Atom ist

auf Pilze, ihren Lebensraum, Saatgüter, Pflanzen, Boden oder Materialien einwirken läßt.

6. Verwendung von Benzyloxyalkylazolverbindungen der Formel I gemäß Anspruch 1 zur Bekämpfung von Pilzen.

7. Verfahren zur Herstellung von Fungiziden, dadurch gekennzeichnet, daß man Benzyloxyalkylazole der Formel I gemäß Anspruch 1 mit festen oder flüssigen Trägerstoffen und/oder oberflächenaktiven Mitteln mischt.

8. Fungizid, enthaltend einen festen oder flüssigen Trägerstoff und ein Benzyloxyalkylazol der allgemeinen Formel I

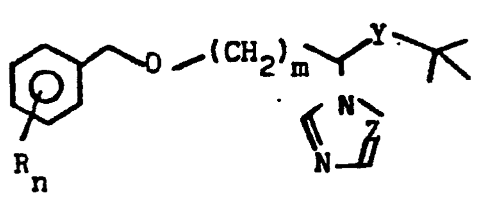

I

in der

R für Halogen, Alkyl, Alkoxy, Halogenalkyl, gegebenenfalls substituiertes Aryl, gegebenenfalls substituiertes Pyridyl, Aralkyl, Alkylthio oder Wasserstoff steht,

n eine ganze Zahl von 1 bis 5 bedeutet,

m eine ganze Zahl von 1 bis 8 bedeutet,

Y für die Gruppen C=O und HCOH steht und

Z eine CH-Gruppe oder ein N-Atom ist.

9. Fungizid gemäß Anspruch 8, dadurch gekennzeichnet, daß R für Wasserstoff, Halogen, niederes Alkyl mit 1 bis 4 Kohlenstoffatomen oder Halogenalkyl mit 1 bis 2 Kohlenstoffatomen und 1 bis 3 Halogenatomen steht.